# EUROPEAN PATENT APPLICATION

(11) **EP 4 647 397 A1**
(43) Date of publication of application: **12.11.2025**
(21) Application number: 23914823.2
(22) Date of filing: 28.12.2023
(51) Int. Cl.: C01B 17/16

(54) **METHOD FOR PRODUCING HYDROGEN SULFIDE AND HYDROGEN SULFIDE PRODUCTION DEVICE**

(30) Priority: 06.01.2023 JP 2023001324
(71) Applicant: Furukawa Co., Ltd., Chiyoda-ku Tokyo 100-8370 (JP)
(72) Inventor: HOSHI Hiroyuki, Tsukuba-shi, Ibaraki 305-0856 (JP)
(74) Representative: Müller, Christian Stefan Gerd
(86) International application number: PCT/JP2023/047269
(87) International publication number: WO 2024/147338

(57) **Abstract**

A method for producing hydrogen sulfide, in which hydrogen sulfide is synthesized by causing sulfur gas and hydrogen gas to react in a reaction tank (101), the method comprising: a step (A) of supplying a mixed gas of sulfur gas and hydrogen gas to the reaction tank (101) ; a step (B) of supplying hydrogen gas to the reaction tank (101); and a step (C) of synthesizing hydrogen sulfide by causing sulfur gas and hydrogen gas to react, wherein an amount of sulfur in the reaction tank (101) is measured, and a supply amount of the hydrogen gas in the step (B) is adjusted based on a result of the measurement.

## Description

### TECHNICAL FIELD

The present invention relates to a method for producing hydrogen sulfide and a hydrogen sulfide producing device.

### BACKGROUND ART

As a method for producing hydrogen sulfide, a method of reacting sulfur gas with hydrogen gas is known.

Examples of the technique related to such a method for producing hydrogen sulfide include the technique described in Patent Document 1.

Patent Document 1 discloses a method for producing hydrogen sulfide in which, in a method for producing hydrogen sulfide by passing gaseous hydrogen and sulfur through a reactor containing a catalyst, a raw material gas is used, the raw material gas being obtained by preparing sulfur vapor containing hydrogen gas in advance and then adding hydrogen gas to the sulfur vapor containing hydrogen gas so that a molar ratio of sulfur atoms to hydrogen molecules is 1 to 1.5. According to the invention described in Patent Document 1, it is described that a method for producing hydrogen sulfide having a low hydrogen gas content can be provided.

### RELATED DOCUMENT

### PATENT DOCUMENT

Patent Document 1: Japanese Unexamined Patent Application, First Publication No. 2003-321212

### SUMMARY OF THE INVENTION

### TECHNICAL PROBLEM

In the conventional method for producing hydrogen sulfide, in a case where the supply of sulfur vapor (sulfur gas) is excessive, a local sulfur excess state occurs in the reaction tank, which may reduce the reaction efficiency.

The present invention has been made in view of the above circumstances, and an object of the present invention is to provide a method for producing hydrogen sulfide and a hydrogen sulfide producing device, which improve the reaction efficiency by suppressing the occurrence of a local sulfur excess state in a reaction tank.

### SOLUTION TO PROBLEM

That is, according to the present invention, a method for producing hydrogen sulfide and a hydrogen sulfide producing device, which will be described below, are provided.
[1] A method for producing hydrogen sulfide, in which hydrogen sulfide is synthesized by causing sulfur gas and hydrogen gas to react in a reaction tank, the method including:
   a step (A) of supplying a mixed gas of sulfur gas and hydrogen gas to the reaction tank;
   a step (B) of supplying hydrogen gas to the reaction tank; and
   a step (C) of synthesizing hydrogen sulfide by causing sulfur gas and hydrogen gas to react,
   in which an amount of sulfur in the reaction tank is measured, and the supply amount of the hydrogen gas in the step (B) is adjusted based on the result of the measurement.
[2] The method for producing hydrogen sulfide according to the above [1], in which, in a case where the amount of sulfur in the reaction tank is excessive, the supply amount of the hydrogen gas in the step (B) is increased.
[3] The method for producing hydrogen sulfide according to the above [1] or [2], in which an internal pressure in the reaction tank is kept constant by adjusting the supply amount of the mixed gas in the step (A) and the supply amount of the hydrogen gas in the step (B).
[4] The method for producing hydrogen sulfide according to any one of the above [1] to [3], in which a porous material is installed in the reaction tank.
[5] The method for producing hydrogen sulfide according to the above [4], in which the porous material includes activated alumina.
[6] The method for producing hydrogen sulfide according to any one of the above [1] to [5], further including a step of heating sulfur in a molten sulfur tank to generate sulfur gas.
[7] The method for producing hydrogen sulfide according to the above [6], in which, in a case where the amount of sulfur in the reaction tank is excessive, a temperature in the molten sulfur tank is decreased.
[8] A hydrogen sulfide producing device that produces hydrogen sulfide by reacting sulfur gas with hydrogen gas, the device including:
   a reaction tank that reacts sulfur gas with hydrogen gas;
   a mixed gas supply unit that supplies a mixed gas of sulfur gas and hydrogen gas;
   a hydrogen gas supply unit that supplies hydrogen gas;
   a sulfur amount measurement unit that measures an amount of sulfur in the reaction tank; and
   a supply adjustment unit that adjusts the supply amount of the mixed gas from the mixed gas supply unit and the supply amount of the hydrogen gas from the hydrogen gas supply unit.
[9] The hydrogen sulfide producing device according to the above [8], in which, in a case where the amount of sulfur in the reaction tank is excessive, a supply amount of the hydrogen gas from the hydrogen gas supply unit is increased.
[10] The hydrogen sulfide producing device according to the above [8] or [9], in which an internal pressure in the reaction tank is kept constant by adjusting a supply amount of the mixed gas and a supply amount of the hydrogen gas.
[11] The hydrogen sulfide producing device according to any one of the above [8] to [10], in which a porous material is installed in the reaction tank.
[12] The hydrogen sulfide producing device according to the above [11], in which the porous material includes activated alumina.
[13] The hydrogen sulfide producing device according to any one of the above [8] to [12], further including: a molten sulfur tank that generates the sulfur gas by heating sulfur.
[14] The hydrogen sulfide producing device according to any one of the above [8] to [13], further including: a sulfur collection unit that collects excessive sulfur gas.
[15] The hydrogen sulfide producing device according to any one of the above [8] to [14], in which the sulfur amount measurement unit is installed in at least one of the inside of the reaction tank or a downstream side of the reaction tank.
[16] The hydrogen sulfide producing device according to any one of the above [8] to [15], in which the sulfur amount measurement unit measures the amount of sulfur by infrared rays.

### ADVANTAGEOUS EFFECTS OF INVENTION

The present invention can provide a method for producing hydrogen sulfide and a hydrogen sulfide producing device, which can suppress an excess state of sulfur in a reaction tank and improve a reaction efficiency.

### BRIEF DESCRIPTION OF THE DRAWINGS

[Fig. 1] A schematic view showing an example of a hydrogen sulfide producing device according to the present invention.
[Fig. 2] A schematic view showing an example of a hydrogen sulfide producing device according to the present invention.
[Fig. 3] A schematic view showing an example of a hydrogen sulfide producing device according to the present invention.
[Fig. 4] A schematic view showing an example of a hydrogen sulfide producing device according to the present invention.
[Fig. 5] A schematic view showing an example of a distal end portion of a sulfur amount measurement unit.
[Fig. 6] A schematic view showing an example of a hydrogen sulfide producing device according to the present invention.
[Fig. 7] A schematic view showing an example of a hydrogen sulfide producing device according to the present invention.
[Fig. 8] A schematic view showing an example of a hydrogen sulfide producing device according to the present invention.
[Fig. 9] A schematic view showing an example of a hydrogen sulfide producing device according to the present invention.
[Fig. 10] A schematic view showing an example of a hydrogen sulfide producing device according to the present invention.
[Fig. 11] A schematic view showing an example of a hydrogen sulfide producing device according to the present invention.

### DESCRIPTION OF EMBODIMENTS

Hereinafter, an embodiment of the present invention will be described with reference to the drawings. In all the drawings, the same components are represented by common reference numerals, and the description thereof will not be repeated. In addition, in the drawings, the shapes, sizes, and arrangement relationships of the respective components are schematically shown to the extent that the present invention can be understood, and are different from the actual sizes.

### [Method for Producing Hydrogen Sulfide]

A method for producing hydrogen sulfide according to the present embodiment is a method for producing hydrogen sulfide, in which hydrogen sulfide is synthesized by causing sulfur gas and hydrogen gas to react in a reaction tank, the method including: a step (A) of supplying a mixed gas of sulfur gas and hydrogen gas to the reaction tank; a step (B) of supplying hydrogen gas to the reaction tank; and a step (C) of synthesizing hydrogen sulfide by causing sulfur gas and hydrogen gas to react, in which an amount of sulfur in the reaction tank is measured, and a supply amount of the hydrogen gas in the step (B) is adjusted based on a result of the measurement.

In a case where a local sulfur excess state occurs in the reaction tank, sulfur is liquefied or solidified at a portion where the sulfur excess state occurs, which inhibits the reaction between sulfur gas and hydrogen gas, and thus the generation efficiency of hydrogen sulfide is lowered. According to the method for producing hydrogen sulfide of the present embodiment, it is possible to suppress the occurrence of a local excess state of sulfur in the reaction tank, and thus it is possible to improve the reaction efficiency.

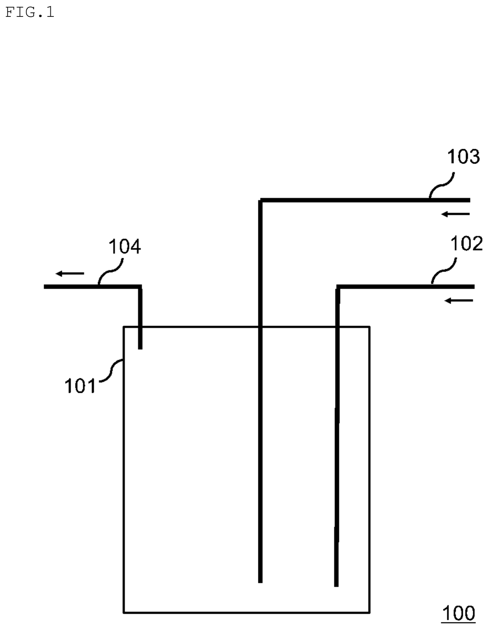

Fig. 1 is a schematic view showing a hydrogen sulfide producing device 100 which is an example of a hydrogen sulfide producing device according to the present invention.

The method for producing hydrogen sulfide according to the present embodiment is a method for producing hydrogen sulfide, in which hydrogen sulfide is synthesized by causing sulfur gas and hydrogen gas to react in a reaction tank 101, the method including: a step (A) of supplying a mixed gas of sulfur gas and hydrogen gas to the reaction tank 101; a step (B) of supplying hydrogen gas to the reaction tank 101; and a step (C) of synthesizing hydrogen sulfide by causing sulfur gas and hydrogen gas to react, in which an amount of sulfur in the reaction tank 101 is measured, and a supply amount of the hydrogen gas in the step (B) is adjusted based on a result of the measurement.

In the step (A), for example, a mixed gas of hydrogen gas and sulfur gas is supplied to the reaction tank 101 by the mixed gas supply unit 102. Examples of the mixed gas supply unit include a pipe.

In the step (B), for example, the hydrogen gas supply unit 103 supplies the hydrogen gas to the reaction tank 101. Examples of the hydrogen gas supply unit include a pipe.

In the step (C), the mixed gas and the hydrogen gas supplied to the reaction tank 101 react with each other in the reaction tank 101, and hydrogen sulfide is synthesized. In the reaction tank 101, it is considered that a reaction represented by Formula (1) occurs.

H₂ + S → H₂S (1)

The generated hydrogen sulfide-containing gas is recovered from the reaction tank 101 by, for example, the hydrogen sulfide-containing gas recovery unit 104. Examples of the hydrogen sulfide-containing gas recovery unit include a pipe.

The reaction tank 101, the mixed gas supply unit 102, the hydrogen gas supply unit 103, and the hydrogen sulfide-containing gas recovery unit 104 include, for example, one or two or more selected from the group consisting of carbon, stainless steel, glass, alumina, aluminum, Inconel (registered trademark), and Hastelloy (registered trademark). From the viewpoint of improving the strength and preventing the mixing of metal impurities, the reaction tank 101 preferably includes one or two or more selected from stainless steel and glass, more preferably includes glass, and still more preferably includes quartz glass.

The reaction tank 101 may be provided with a stirring function. Examples of the stirring function include a rotary kiln type stirring function.

In the method for producing hydrogen sulfide according to the present embodiment, in a case where the amount of sulfur in the reaction tank 101 is excessive, it is preferable to increase the supply amount of the hydrogen gas in the step (B). As a result, the reaction between the excess sulfur and the hydrogen gas is promoted, and the generation of a local excess state of sulfur in the reaction tank can be further suppressed.

In the method for producing hydrogen sulfide according to the present embodiment, it is preferable that the internal pressure in the reaction tank 101 is kept constant by adjusting the supply amount of the mixed gas in the step (A) and the supply amount of the hydrogen gas in the step (B). As a result, it is possible to suppress a change in reaction conditions due to a change in the internal pressure in the reaction tank 101.

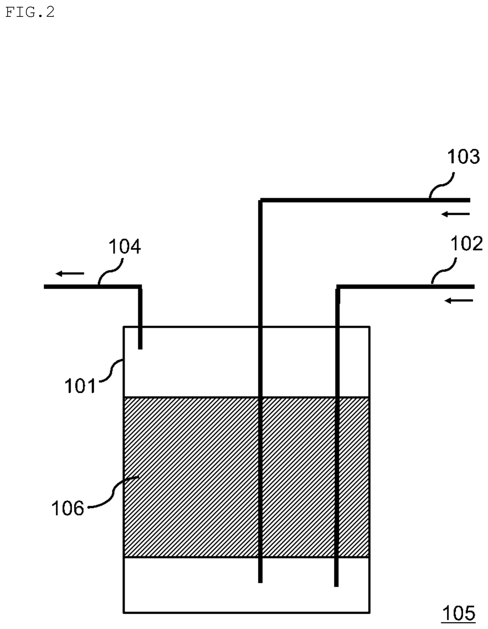

Fig. 2 is a schematic view showing a hydrogen sulfide producing device 105 which is an example of a hydrogen sulfide producing device according to the embodiment of the present invention.

From the viewpoint of promoting the generation reaction of hydrogen sulfide, a porous material 106 is preferably installed in the reaction tank 101. As a result, the reaction between the sulfur gas and the hydrogen gas is promoted on the surface of the porous material 106, and thus the synthesis of hydrogen sulfide is further promoted.

From the viewpoint of promoting the generation reaction of hydrogen sulfide, the porous material 106 includes one or two or more materials selected from the group consisting of activated carbon, zeolite, and activated alumina, and more preferably includes activated alumina.

From the viewpoint of promoting the generation of hydrogen sulfide, the pores of the porous material 106 preferably support one or two or more metal elements selected from the group consisting of silver, platinum, molybdenum, cobalt, nickel, iron, and vanadium.

The temperature at the time of generating hydrogen sulfide, that is, the temperature of the step (C) is preferably 300°C or higher, more preferably 330°C or higher, and still more preferably 360°C or higher from the viewpoint of promoting the generation of hydrogen sulfide, and is preferably 500°C or lower, more preferably 480°C or lower, and still more preferably 450°C or lower from the viewpoint of suppressing a side reaction and deterioration of a catalyst.

The heating device in the step (C) is not particularly limited, and is composed of, for example, a heating unit that can heat the inside of the reaction tank 101 and a temperature controller that can adjust the output of the heating unit to maintain the inside of the reaction tank 101 at a constant temperature. The heating unit is not particularly limited, and known heating units such as a heating wire and lamp heating can be used. Any heating unit that can heat the inside of the reaction tank 101 may be used.

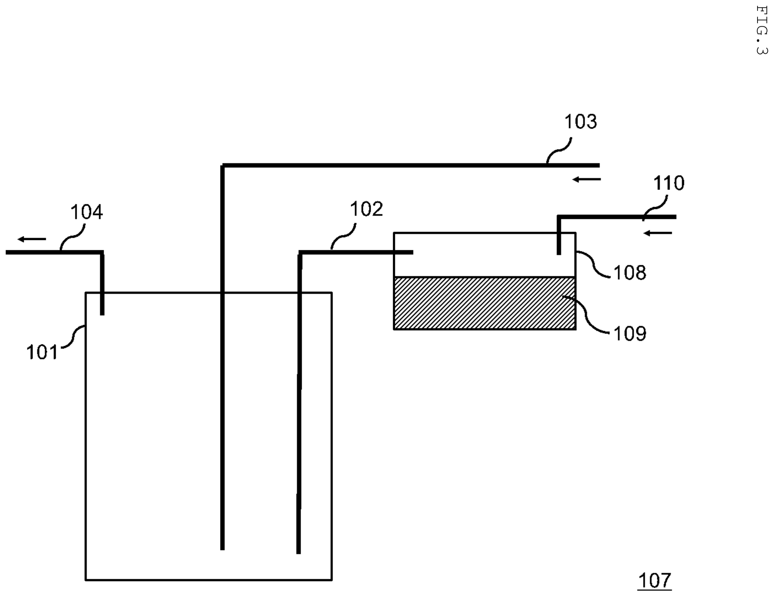

Fig. 3 is a schematic view showing a hydrogen sulfide producing device 107 which is an example of a hydrogen sulfide producing device according to the present invention.

The method for producing hydrogen sulfide according to the present embodiment preferably further includes a step of heating sulfur 109 in the molten sulfur tank 108 to generate sulfur gas. As a result, the supply amount of the sulfur gas is easily controlled.

In a case where hydrogen is supplied to the molten sulfur tank 108 from the hydrogen gas supply unit 110, the sulfur gas generated in the molten sulfur tank 108 and the hydrogen gas supplied from the hydrogen gas supply unit 110 are mixed, and a mixed gas of the sulfur gas and the hydrogen gas is obtained. The mixed gas is supplied from the mixed gas supply unit 102 to the reaction tank 101.

The temperature of the molten sulfur tank 108 is not particularly limited as long as the temperature is a temperature at which sulfur vapor is generated, and is, for example, 180°C or higher, preferably 220°C or higher, more preferably 260°C or higher, still more preferably 280°C or higher, and even still more preferably 300°C or higher, and is, for example, 440°C or lower, preferably 400°C or lower, more preferably 360°C or lower, still more preferably 340°C or lower, and even still more preferably 320°C or lower, from the viewpoint of suppressing the generation of rubber-like sulfur.

In the method for producing hydrogen sulfide according to the present embodiment, in a case where the amount of sulfur in the reaction tank 101 is excessive, it is preferable to reduce the temperature in the molten sulfur tank 108. As a result, since the amount of sulfur gas generated in the molten sulfur tank 108 is reduced, the supply amount of the sulfur gas to the reaction tank 101 can be reduced, and as a result, the occurrence of a local excess state of sulfur in the reaction tank can be further suppressed.

### [Hydrogen Sulfide Producing Device]

A hydrogen sulfide producing device according to the present embodiment is a hydrogen sulfide producing device that produces hydrogen sulfide by reacting sulfur gas with hydrogen gas, the device including:
a reaction tank that reacts sulfur gas with hydrogen gas;
a mixed gas supply unit that supplies a mixed gas of sulfur gas and hydrogen gas;
a hydrogen gas supply unit that supplies hydrogen gas;
a sulfur amount measurement unit that measures an amount of sulfur in the reaction tank; and
a supply adjustment unit that adjusts a supply amount of the mixed gas from the mixed gas supply unit and a supply amount of the hydrogen gas from the hydrogen gas supply unit.

In a case where a local sulfur excess state occurs in the reaction tank, sulfur is liquefied or solidified at a portion where the sulfur excess state occurs, which inhibits the reaction between sulfur gas and hydrogen gas, and thus the generation efficiency of hydrogen sulfide is lowered.

With the hydrogen sulfide producing device according to the present embodiment, the amount of sulfur in the reaction tank can be measured by the sulfur amount measurement unit. Further, according to the supply adjustment unit, it is possible to adjust the supply amount of the mixed gas and the supply amount of the hydrogen gas according to the measured sulfur amount.

For example, in a case where the measured sulfur amount is excessive, the supply adjustment unit can increase the supply amount of hydrogen gas by operating the supply adjustment unit, and thus the occurrence of a local excess state of sulfur in the reaction tank can be suppressed, whereby the reaction efficiency can be improved.

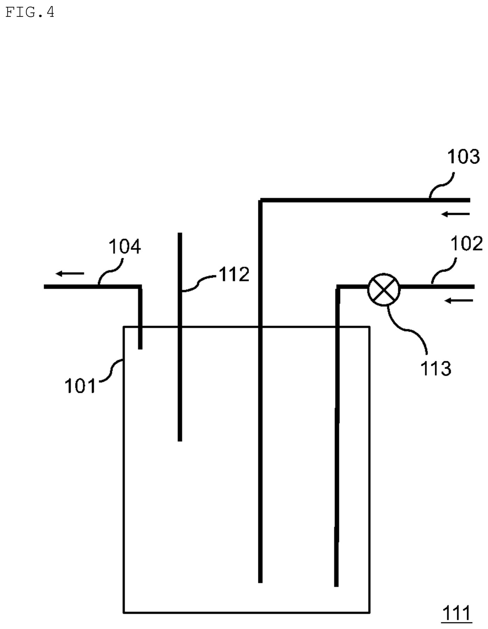

Fig. 4 is a schematic view showing a hydrogen sulfide producing device 111 which is an example of a hydrogen sulfide producing device according to the embodiment of the present invention.

A hydrogen sulfide producing device 111 according to the present embodiment is a hydrogen sulfide producing device that produces hydrogen sulfide by reacting sulfur gas with hydrogen gas, and includes a reaction tank 101 that reacts sulfur gas with hydrogen gas, a mixed gas supply unit 102 that supplies a mixed gas of sulfur gas and hydrogen gas, a hydrogen gas supply unit 103 that supplies hydrogen gas, a sulfur amount measurement unit 112 that measures an amount of sulfur in the reaction tank 101, and a supply adjustment unit 113 that adjusts a supply amount of the mixed gas from the mixed gas supply unit 102 and a supply amount of the hydrogen gas from the hydrogen gas supply unit 103.

Examples of the mixed gas supply unit 102 and the hydrogen gas supply unit 103 include a pipe.

According to the hydrogen sulfide producing device 111, a mixed gas of hydrogen gas and sulfur gas is supplied to the reaction tank 101 by the mixed gas supply unit 102, and hydrogen gas is supplied to the reaction tank 101 by the hydrogen gas supply unit 103.

The mixed gas and the hydrogen gas supplied to the reaction tank 101 react in the reaction tank 101 to generate hydrogen sulfide. In the reaction tank 101, it is considered that a reaction represented by Formula (1) occurs.

H₂ + S → H₂S (1)

The generated hydrogen sulfide-containing gas is recovered from the reaction tank 101 by, for example, the hydrogen sulfide-containing gas recovery unit 104. Examples of the hydrogen sulfide-containing gas recovery unit 104 include a pipe.

The reaction tank 101, the mixed gas supply unit 102, the hydrogen gas supply unit 103, and the hydrogen sulfide-containing gas recovery unit 104 include, for example, one or two or more selected from the group consisting of carbon, stainless steel, glass, alumina, aluminum, Inconel (registered trademark), and Hastelloy (registered trademark). From the viewpoint of improving the strength and preventing the mixing of metal impurities, the reaction tank 101 preferably includes one or two or more selected from stainless steel and glass, more preferably includes glass, and still more preferably includes quartz glass.

The reaction tank 101 may be provided with a stirring function. Examples of the stirring function include a rotary kiln type stirring function.

The sulfur amount measurement unit 112 measures the amount of sulfur in the reaction tank 101.

It is preferable that the sulfur amount measurement unit 112 is installed in at least one of the inside of the reaction tank 101 or the downstream side of the reaction tank 101. As a result, it is possible to measure the amount of sulfur that is excessive during the hydrogen sulfide generation reaction or after the hydrogen sulfide generation reaction.

Examples of the sulfur amount measurement unit 112 include a sensor capable of measuring the amount of sulfur.

The sulfur amount measurement unit 112 preferably measures the amount of sulfur by infrared rays.

Specifically, the amount of sulfur can be measured by infrared rays according to the following <Method>.

### <Method>

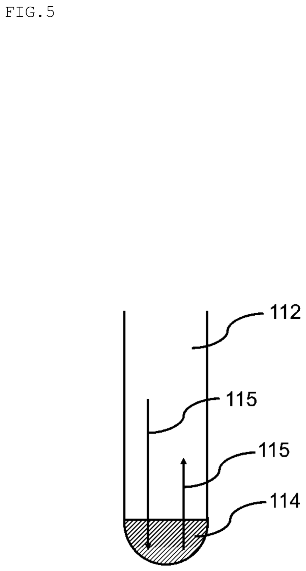

(1) A quartz glass rod as the sulfur amount measurement unit 112 is inserted into the reaction tank 101 from the upper portion of the reaction tank 101.
(2) Hereinafter, a schematic view in which the distal end portion of the quartz glass rod is shown in an enlarged view will be described with reference to Fig. 5. In a case where the hydrogen sulfide generation reaction is performed in a state where the quartz glass rod is inserted, a liquid droplet 114 of sulfur adheres to the distal end of the quartz glass rod.
(3) The liquid droplet 114 is irradiated with infrared rays 115 through the quartz glass rod.
(4) A part of the infrared rays 115 is absorbed by the liquid droplet 114, and a part thereof is reflected.
(5) The amount of the reflected infrared ray 115 is measured, and the absorption amount of the infrared ray 115 absorbed by the liquid droplet 114 is calculated from the value.
(6) Since the absorption amount of the infrared ray 115 is proportional to the thickness of the liquid droplet 114, the thickness of the liquid droplet 114 is calculated from the absorption amount of the infrared ray 115.
(7) The sulfur excess state is detected from the thickness of the liquid droplet 114.

Examples of the supply adjustment unit 113 include a valve.

The supply adjustment unit 113 can adjust the supply amount of the mixed gas and the supply amount of the hydrogen gas. In the hydrogen sulfide producing device 111, a supply adjustment unit 113 is provided in the mixed gas supply unit 102. In a case where the supply adjustment unit 113 is opened, the supply amount of the mixed gas increases, and the supply amount of the hydrogen gas relatively decreases. On the other hand, in a case where the supply adjustment unit 113 is closed, the supply amount of the mixed gas is decreased, and the supply amount of the hydrogen gas is relatively increased. In this way, the supply amount of the hydrogen gas can be adjusted.

In the hydrogen sulfide producing device according to the present embodiment, in a case where the amount of sulfur in the reaction tank 101 is excessive, it is preferable to increase the supply amount of the hydrogen gas. As a result, the reaction between the excess sulfur and the hydrogen gas is promoted, and the generation of a local excess state of sulfur in the reaction tank can be further suppressed. A method of increasing the supply amount of the hydrogen gas is not particularly limited, but for example, the supply amount of the hydrogen gas can be relatively increased by closing the supply adjustment unit 113 provided in the mixed gas supply unit 102 to decrease the supply amount of the mixed gas.

In the hydrogen sulfide producing device according to the present embodiment, it is preferable that the internal pressure in the reaction tank is kept constant by adjusting the supply amount of the mixed gas and the supply amount of the hydrogen gas. As a result, it is possible to suppress a change in reaction conditions due to a change in the internal pressure in the reaction tank 101.

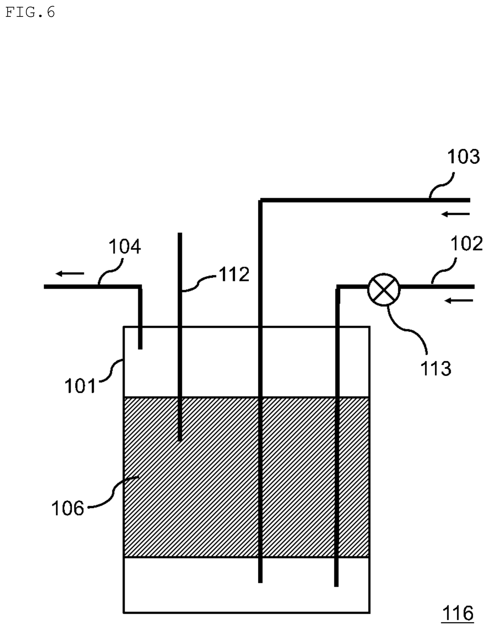

Fig. 6 is a schematic view showing a hydrogen sulfide producing device 116 which is an example of a hydrogen sulfide producing device according to the embodiment of the present invention.

A porous material 106 is preferably installed in the reaction tank 101. In a case where the porous material 106 is installed in the reaction tank 101, the sulfur gas and the hydrogen gas react on the surface of the porous material 106, and hydrogen sulfide is generated. As a result, the reaction between the sulfur gas and the hydrogen gas is promoted on the surface of the porous material 106, and thus the synthesis of hydrogen sulfide is further promoted.

From the viewpoint of promoting the generation reaction of hydrogen sulfide, the porous material 106 includes, for example, one or two or more materials selected from the group consisting of activated carbon, zeolite, and activated alumina, and more preferably includes activated alumina.

From the viewpoint of promoting the generation of hydrogen sulfide, the pores of the porous material 106 preferably support one or two or more metal elements selected from the group consisting of silver, platinum, molybdenum, cobalt, nickel, iron, and vanadium.

The temperature at the time of generating hydrogen sulfide is preferably 300°C or higher, more preferably 330°C or higher, and still more preferably 360°C or higher from the viewpoint of promoting the generation of hydrogen sulfide, and is preferably 500°C or lower, more preferably 480°C or lower, and still more preferably 450°C or lower from the viewpoint of suppressing a side reaction and deterioration of a catalyst.

The heating device at the time of generating hydrogen sulfide is not particularly limited, and is composed of, for example, a heating unit that can heat the inside of the reaction tank 101 and a temperature controller that can adjust the output of the heating unit to maintain the inside of the reaction tank 101 at a constant temperature. The heating unit is not particularly limited, and known heating units such as a heating wire and a lamp heating can be used. Any heating unit that can heat the inside of the reaction tank 101 may be used.

It is preferable that the mixed gas and the hydrogen gas are supplied from a portion below the portion of the reaction tank 101 in which the porous material 106 is accommodated. As a result, as the hydrogen gas lighter than air rises in the reaction tank 101, an upward current is generated in the reaction tank 101. Due to the upward current, the sulfur gas and the hydrogen gas are well in contact with the porous material 106, and the reaction efficiency is further improved.

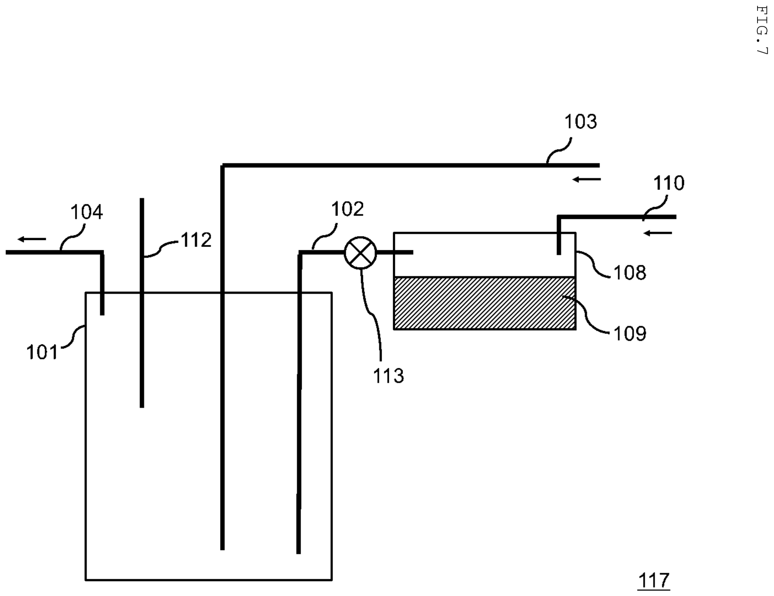

Fig. 7 is a schematic view showing a hydrogen sulfide producing device 117 which is an example of a hydrogen sulfide producing device according to the embodiment of the present invention.

It is preferable that the hydrogen sulfide producing device according to the present embodiment further includes a molten sulfur tank 108 in which sulfur 109 is heated to generate sulfur gas. As a result, the supply amount of the sulfur gas is easily controlled.

In a case where hydrogen is supplied to the molten sulfur tank 108 from the hydrogen gas supply unit 110, the sulfur gas generated in the molten sulfur tank 108 and the hydrogen gas supplied from the hydrogen gas supply unit 110 are mixed, and a mixed gas of the sulfur gas and the hydrogen gas is obtained. The obtained mixed gas is supplied from the mixed gas supply unit 102 to the reaction tank 101.

The temperature of the molten sulfur tank 108 is not particularly limited as long as the temperature is a temperature at which sulfur vapor is generated, and is, for example, 180°C or higher, preferably 220°C or higher, more preferably 260°C or higher, still more preferably 280°C or higher, and even still more preferably 300°C or higher, and is, for example, 440°C or lower, preferably 400°C or lower, more preferably 360°C or lower, still more preferably 340°C or lower, and even still more preferably 320°C or lower, from the viewpoint of suppressing the generation of rubber-like sulfur.

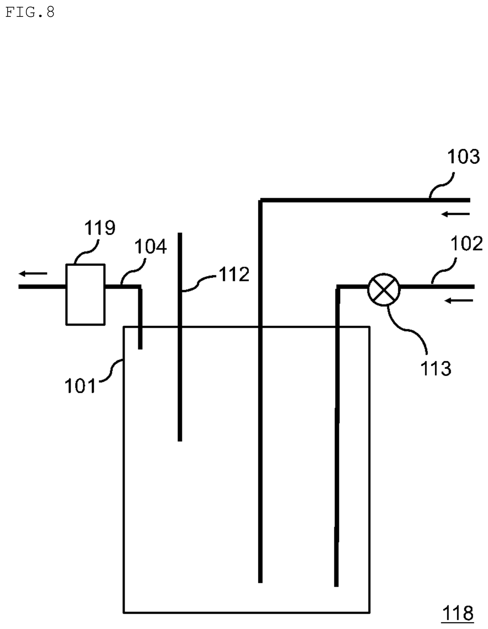

Fig. 8 is a schematic view showing a hydrogen sulfide producing device 118 which is an example of a hydrogen sulfide producing device according to the embodiment of the present invention.

The hydrogen sulfide producing device according to the present embodiment preferably further includes a sulfur collection unit 119 that collects excess sulfur gas.

Examples of a method of collecting the excess sulfur by the sulfur collection unit 119 include a method of cooling the hydrogen sulfide-containing gas recovered from the hydrogen sulfide-containing gas recovery unit 104 by the sulfur collection unit 119 and liquefying or solidifying the hydrogen sulfide-containing gas to collect the excess sulfur contained in the gas.

It is also possible to measure the amount of sulfur in the reaction tank 101 from the amount of sulfur collected in the sulfur collection unit 119.

For example, the amount of sulfur in the reaction tank 101 may be calculated from the weight of the sulfur collected in the sulfur collection unit 119.

In addition, a glass window may be provided in the sulfur collection unit 119, and the amount of sulfur collected in the sulfur collection unit 119 may be calculated from the value of the light transmittance of the glass window, and the amount of sulfur in the reaction tank 101 may be calculated from the calculated amount of sulfur. As the amount of sulfur in the reaction tank 101 increases, the amount of sulfur collected in the sulfur collection unit 119 increases, and thus the amount of sulfur adhering to the glass window increases, which results in a decrease in the light transmittance of the glass window.

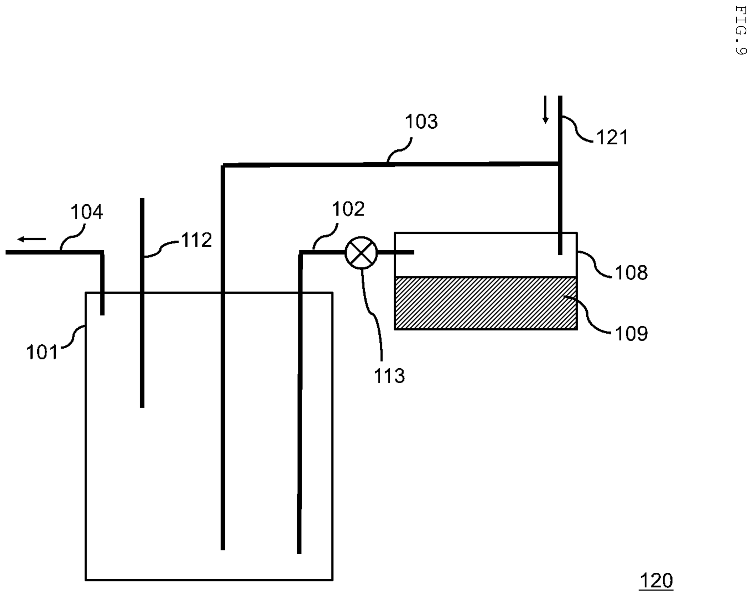

Fig. 9 is a schematic view showing a hydrogen sulfide producing device 120 which is an example of a hydrogen sulfide producing device according to the embodiment of the present invention.

In the hydrogen sulfide producing device according to the present embodiment, for example, the supply of the mixed gas and the hydrogen gas can be switched by opening and closing the supply adjustment unit 113.

In a case where the supply adjustment unit 113 is opened, the hydrogen gas supplied from the hydrogen gas supply unit 121 is supplied to the molten sulfur tank 108, and a mixed gas of the hydrogen gas and the sulfur gas is obtained there. The mixed gas is supplied from the mixed gas supply unit 102 to the reaction tank 101.

On the other hand, in a case where the supply adjustment unit 113 is closed, the hydrogen gas supplied from the hydrogen gas supply unit 121 is supplied to the reaction tank 101 via the hydrogen gas supply unit 103.

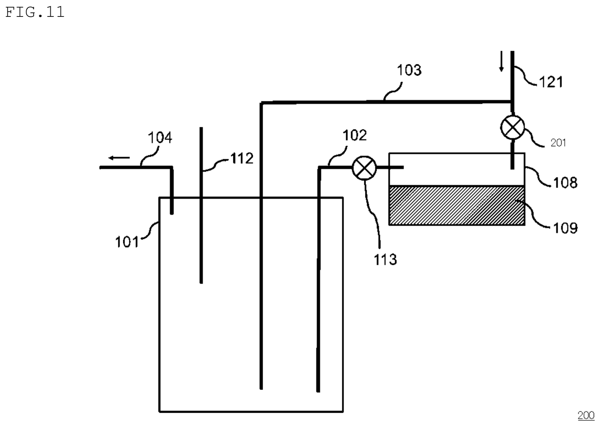

The hydrogen sulfide producing device 120 may include an embodiment such as a hydrogen sulfide producing device 200 shown in the schematic view of Fig. 11. The hydrogen sulfide producing device 200 is an example of a hydrogen sulfide producing device according to the present invention.

In the hydrogen sulfide producing device 200, for example, a hydrogen gas supply adjustment unit 201 is provided between the molten sulfur tank 108 and the hydrogen gas supply unit 121. The hydrogen gas supply adjustment unit 201 adjusts the supply amount of the hydrogen gas from the hydrogen gas supply unit 121. Examples of the hydrogen gas supply adjustment unit 201 include a valve with a check valve.

The supply amount of the hydrogen gas supplied to the molten sulfur tank 108 can be adjusted by the hydrogen gas supply adjustment unit 201. In the hydrogen sulfide producing device 200, for example, a hydrogen gas supply adjustment unit 201 is provided between a portion where the hydrogen gas supply unit 103 branches off from the hydrogen gas supply unit 121 and the molten sulfur tank 108. In a case where the hydrogen gas supply adjustment unit 201 is opened, the hydrogen gas can be supplied from the hydrogen gas supply unit 121 into the molten sulfur tank 108. On the other hand, in a case where the hydrogen gas supply adjustment unit 201 is closed, the hydrogen gas supply adjustment unit 201 can stop the supply of the hydrogen gas from the hydrogen gas supply unit 121 into the molten sulfur tank 108 and can reduce the backflow of the sulfur gas from the molten sulfur tank 108 to the hydrogen gas supply unit 121 and the hydrogen gas supply unit 103.

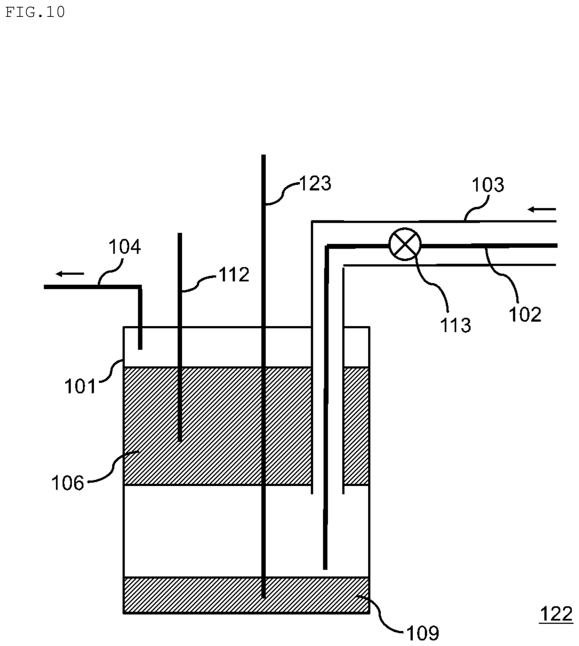

Fig. 10 is a schematic view showing a hydrogen sulfide producing device 122 which is an example of a hydrogen sulfide producing device according to the embodiment of the present invention.

In the hydrogen sulfide producing device according to the present embodiment, for example, sulfur 109 is heated in a lower portion of the reaction tank 101 to generate sulfur gas.

In the hydrogen sulfide producing device according to the present embodiment, for example, a sulfur supply unit 123 for supplying sulfur to the reaction tank 101 is provided, and in a case where sulfur is insufficient during the hydrogen sulfide generation reaction, sulfur is supplied from the sulfur supply unit 123.

In the hydrogen sulfide producing device according to the present embodiment, for example, the mixed gas supply unit 102 and the hydrogen gas supply unit 103 form a double pipe structure.

In the hydrogen sulfide producing device according to the present embodiment, for example, it is preferable that the mixed gas supply unit 102 is positioned inside the double pipe structure, the terminal end of the mixed gas supply unit 102 is positioned near the liquid surface of the sulfur 109, and the hydrogen gas is supplied from the terminal end of the mixed gas supply unit 102. Here, a mechanism in which the mixed gas is supplied to the reaction tank 101 by the mixed gas supply unit 102 will be described. First, the hydrogen gas supplied from the terminal end of the mixed gas supply unit 102 raises the sulfur gas generated near the liquid surface of the sulfur 109. Then, the hydrogen gas and the sulfur gas are mixed to obtain a mixed gas. Then, the mixed gas obtained in this way is supplied to the reaction tank 101. That is, the hydrogen gas is supplied from the terminal end of the mixed gas supply unit 102, but the hydrogen gas supplied from the terminal end of the mixed gas supply unit 102 raises the sulfur gas, and as a result, the mixed gas is supplied to the reaction tank 101.

In the hydrogen sulfide producing device according to the present embodiment, for example, it is preferable that the mixed gas supply unit 102 is movable in the vertical direction. As a result, even in a case where the position of the liquid surface of the sulfur 109 fluctuates, it is possible to adjust the position of the terminal end of the mixed gas supply unit 102 in accordance with the fluctuation. As a result, the entrainment of sulfur gas can be performed more efficiently.

In the hydrogen sulfide producing device according to the present embodiment, for example, it is preferable that the hydrogen gas supply unit 103 is positioned outside the double pipe structure, the terminal end of the hydrogen gas supply unit 103 is positioned near the lower end of the porous material 106, and a sufficient distance is maintained between the lower end of the porous material 106 and the liquid surface of the sulfur 109. As a result, the hydrogen gas supplied from the hydrogen gas supply unit 103 rises in the reaction tank 101 without raising up the sulfur gas. In this way, the hydrogen gas is supplied from the hydrogen gas supply unit 103 to the reaction tank 101.

This application claims priority based on Japanese Patent Application No. 2023-001324 filed on January 6, 2023, the entire disclosure of which is incorporated herein by reference.

### REFERENCE SIGNS LIST

- 100: hydrogen sulfide producing device
- 101: reaction tank
- 102: mixed gas supply unit
- 103: hydrogen gas supply unit
- 104: hydrogen sulfide-containing gas recovery unit
- 105: hydrogen sulfide producing device
- 106: porous material
- 107: hydrogen sulfide producing device
- 108: molten sulfur tank
- 109: sulfur
- 110: hydrogen gas supply unit
- 111: hydrogen sulfide producing device
- 112: sulfur amount measurement unit
- 113: supply adjustment unit
- 114: liquid droplet
- 115: infrared ray
- 116: hydrogen sulfide producing device
- 117: hydrogen sulfide producing device
- 118: hydrogen sulfide producing device
- 119: sulfur collection unit
- 120: hydrogen sulfide producing device
- 121: hydrogen gas supply unit
- 122: hydrogen sulfide producing device
- 123: sulfur supply unit
- 200: hydrogen sulfide producing device
- 201: hydrogen gas supply adjustment unit

## Claims

1. A method for producing hydrogen sulfide, in which hydrogen sulfide is synthesized by causing sulfur gas and hydrogen gas to react in a reaction tank, the method comprising:
a step (A) of supplying a mixed gas of sulfur gas and hydrogen gas to the reaction tank;
a step (B) of supplying hydrogen gas to the reaction tank; and
a step (C) of synthesizing hydrogen sulfide by causing sulfur gas and hydrogen gas to react,
wherein an amount of sulfur in the reaction tank is measured, and the supply amount of the hydrogen gas in the step (B) is adjusted based on the result of the measurement.

2. The method for producing hydrogen sulfide according to Claim 1,
wherein, in a case where the amount of sulfur in the reaction tank is excessive, the supply amount of the hydrogen gas in the step (B) is increased.

3. The method for producing hydrogen sulfide according to Claim 1 or 2,
wherein an internal pressure in the reaction tank is kept constant by adjusting the supply amount of the mixed gas in the step (A) and the supply amount of the hydrogen gas in the step (B) .

4. The method for producing hydrogen sulfide according to any one of Claims 1 to 3,
wherein a porous material is installed in the reaction tank.

5. The method for producing hydrogen sulfide according to Claim 4,
wherein the porous material includes activated alumina.

6. The method for producing hydrogen sulfide according to any one of Claims 1 to 5, further comprising:
a step of heating sulfur in a molten sulfur tank to generate sulfur gas.

7. The method for producing hydrogen sulfide according to Claim 6,
wherein, in a case where the amount of sulfur in the reaction tank is excessive, a temperature in the molten sulfur tank is decreased.

8. A hydrogen sulfide producing device that produces hydrogen sulfide by reacting sulfur gas with hydrogen gas, the device comprising:
a reaction tank that reacts sulfur gas with hydrogen gas;
a mixed gas supply unit that supplies a mixed gas of sulfur gas and hydrogen gas;
a hydrogen gas supply unit that supplies hydrogen gas;
a sulfur amount measurement unit that measures an amount of sulfur in the reaction tank; and
a supply adjustment unit that adjusts the supply amount of the mixed gas from the mixed gas supply unit and the supply amount of the hydrogen gas from the hydrogen gas supply unit.

9. The hydrogen sulfide producing device according to Claim 8,
wherein, in a case where the amount of sulfur in the reaction tank is excessive, a supply amount of the hydrogen gas from the hydrogen gas supply unit is increased.

10. The hydrogen sulfide producing device according to Claim 8 or 9,
wherein an internal pressure in the reaction tank is kept constant by adjusting a supply amount of the mixed gas and a supply amount of the hydrogen gas.

11. The hydrogen sulfide producing device according to any one of Claims 8 to 10,
wherein a porous material is installed in the reaction tank.

12. The hydrogen sulfide producing device according to Claim 11,
wherein the porous material includes activated alumina.

13. The hydrogen sulfide producing device according to any one of Claims 8 to 12, further comprising:
a molten sulfur tank that generates the sulfur gas by heating sulfur.

14. The hydrogen sulfide producing device according to any one of Claims 8 to 13, further comprising:
a sulfur collection unit that collects excessive sulfur gas.

15. The hydrogen sulfide producing device according to any one of Claims 8 to 14,
wherein the sulfur amount measurement unit is installed in at least one of the inside of the reaction tank or a downstream side of the reaction tank.

16. The hydrogen sulfide producing device according to any one of Claims 8 to 15,
wherein the sulfur amount measurement unit measures the amount of sulfur by infrared rays.
